(19) **Europäisches Patentamt / European Patent Office / Office européen des brevets**

(11) **EP 4 782 016 A1**

(12) # EUROPEAN PATENT APPLICATION

(43) Date of publication:
**29.07.2026  Bulletin 2026/31**

(21) Application number: **25153704.9**

(22) Date of filing: **24.01.2025**

(51) International Patent Classification (IPC):
**A61L 27/04** (2006.01)  **A61L 27/22** (2006.01)
**A61L 27/34** (2006.01)  **A61L 31/02** (2006.01)
**A61L 31/04** (2006.01)  **A61L 31/10** (2006.01)

(52) Cooperative Patent Classification (CPC):
**A61L 27/047; A61L 27/227; A61L 27/34;
A61L 31/022; A61L 31/043; A61L 31/10**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Fibrothelium GmbH
52068 Aachen (DE)**

(72) Inventors:
• **Dr. Kopp, Alexander**
  **52066 Aachen (DE)**
• **Leemhuis Company, Hans**
  **52062 Aachen (DE)**
• **Isella, Benedetta**
  **52062 Aachen (DE)**

(74) Representative: **Bungartz, Florian
Van-Gogh-Strasse 3
81479 München (DE)**

(54) **MEDICAL IMPLANT AND METHOD FOR THE PRODUCTION THEREOF**

(57)    A medical implant comprising a tubular first component, which is partially or entirely made of isolated Bombyx mori fibroin, and at least a second component, whereas the second component has a less compact structure compared to the first component.

## Fig. 2

Teflon Substrate → Dip-coating → Drying → WVA vacuum → Removal → Electro-spinning → WVA 45°C

30°C    Vacuum

Repeated for 8 layers

45°C

**Description**

FIELD OF THE INVENTION

The invention relates to a medical implant and to a method for the production thereof.

Medical implants are widely used in the healthcare industry for various applications such as screws, plates, stents, grafts, and other devices that are placed inside or on the surface of the body. Many of these implants are tubular structures that are used as intraluminal or extraluminal devices in various body systems and compartments to treat diseases or injuries.

BACKGROUND OF THE INVENTION

Endoluminal and extraluminal tubular structures play a crucial role in modern medical practices, offering vital solutions for the diagnosis, treatment, and management of various medical conditions. These structures, which encompass devices such as stents, patches, suture-less anastomosis devices, and grafts, are instrumental in navigating, supporting, or enhancing the function of the body's systems, such as blood vessels, airways, urinary tracts, lymphatic vessels, nerves, tendons, ligaments, bones, and gastrointestinal tracts. Their applications span a wide range of medical specialties, including cardiology, interventional radiology, orthopedy, pulmonology, gastroenterology, urology, transplant surgery, and vascular surgery. Endoluminal devices, inserted into the body's natural lumens, facilitate minimally invasive procedures that reduce patient recovery time and the risk of complications. Meanwhile, extraluminal devices, placed outside the luminal structures, serve to support or protect surrounding tissues and organs. Together, these tubular structures serve to provide significant advancements in patient care, enabling less invasive interventions, improved outcomes, and a higher quality of life for patients worldwide. However, in the current clinical practice, these devices are made of non-(bio)absorbable synthetic materials exposing the patients to the necessity of reinterventions for the removal or the migration of the device, to late side-effects such as restenosis due to an excessive inflammatory reaction or to the mismatch in mechanical properties between the luminal devices and the physiological structures, and to the risk of accumulation of nonself residues which could be potentially toxic and could overload organs such as liver and kidneys. For example, the biliary reconstruction accounts for 40 - 60 % of complications in liver transplants, with bile leaks and strictures leading to severe outcomes as increased morbidity and mortality. Current stenting solutions, typically fabricated from synthetic, non-bioabsorbable materials, necessitate secondary surgical removal, imposing additional risks and healthcare costs. Similarly, vascular grafts and small-diameter vascular grafts used in coronary bypass surgeries face complications from compliance mismatches, thrombogenicity, and immune responses associated with synthetic materials. This lack of suitable off-the-shelf vascular grafts is further complicated by the generalized character of the pathologies leading to coronary bypass and so to the lack of suitable autografts in approximately 30 % of the patients, especially in case of reintervention. Also, in the case of oesophageal stents, the existing solutions for recurrent benign strictures are currently non-bioabsorbable requiring a second intervention for their removal and exposing the patients to the risk of tissue hyperplasia, restenosis, and stent migration. The tubular structures need also to be adaptable in size and shape to the organ or tissue they need to be applied to, preventing at the same time the leakage of fluids without completely occluding the system. For example, the use of flared ends could be beneficial in the design of oesophageal and biliary stents to prevent their migration, while the presence of longitudinal holes could be particularly effective to enhance the passage of fluid in the case of ureteral stents. There is therefore a strong need to optimise the materials used in these tubular structures and the technologies used to manufacture them in order to improve the performance of such devices.

US 9,808,557 B2 discloses silk fibroin compositions and methods for their manufacture and use. The tubular compositions can be used in the repair or replacement of damaged or diseased blood vessels.

This patent addresses innovations in the design, application, and fabrication of such tubular systems, aiming to further enhance their safety, efficiency, and effectiveness in medical practice.

SUMMARY OF THE INVENTION

The objective of the invention is to provide a tubular medical implant, which has at least one advantage over the tubular medical implants according to the prior art.

This objective is achieved by a tubular medical implant as claimed in claim 1. A preferred method for producing such a tubular medical implant is the subject of claim 12. Preferred uses of the medical implant are claimed in claim 15. Preferred embodiments of the medical implant and its production method are subject of the additional claims and are further disclosed in the following description and the appendant drawings.

DETAILS OF THE INVENTION

The invention relates to a medical implant comprising a tubular first component, made partially or entirely of isolated Bombyx mori fibroin, and a second component. The first component and the second component may be structurally separated from each other or they may merge into one another.

Bombyx mori fibroin is a natural protein derived from the silk of the silkworm Bombyx mori. This silk in an original/natural form also comprises sericin as a protein. However, the fibroin of the medical implant according to the invention is isolated, which means that it has been separated from the sericin with which it was combined in the original silk. Thus, the isolated fibroin is at least (as much as possible) free of sericin. Separating the fibroin of the sericin of Bombyx mori silk may generally be achieved by a degumming procedure usually performed in Sodium Carbonate ($Na_2CO_3$) which is then followed by a step of dissolution, usually performed in Lithium Bromide (LiBr), in which the protein fibroin is dispersed into the solvent which is then removed by a dialysis process. A suitable method for producing isolated fibroin in a solution is disclosed in WO 2023/099382 A1, the content of which is incorporated into this specification by reference.

The Bombyx mori fibroin, additional to being isolated, may be purified. Purification may be achieved by a post-treatment of the obtained silk fibroin solution which allows the removal of endotoxins.

Bombyx mori fibroin as used in the first component is biocompatible and absorbable and has good mechanical properties, making it an advantageous material for medical implants.

In the context of this invention the term "absorbable" is regarded as being synonymous with the terms "bioabsorbable", "degradable", "biodegradable", "resorbable" and "bioresorbable".

The tubular first component, made of fibroin, provides strength, flexibility, bioactivity and biocompatibility, ensuring the implant can withstand physical stresses, will not trigger an adverse immune response and supports tissue regeneration.

The Bombyx mori fibroin used for these tubular structures may be purified through the reduction of the pH as described in WO 2023/099382 A1 to reduce the endotoxin level which may be then lower than 1 IU/ml and with a bioburden level lower than 5 CFU/ml. Other elemental impurities measured through ICP may not account for more than 50 ppm. The Bombyx mori fibroin may have a broad molecular weight distribution as measured with gel permeation chromatography, but the curve should preferably be centred in 100 kDa, 150 kDa, 200 kDa, or 250 kDa. The pH value should preferably be neutral with a crystalline β-sheet content superior to 30 %.

The implant also includes a second component with a less compact structure than the first component. "Compact" in the context of the invention relates to the content of voids in the structure of the different components of the medical implant. Thus, the structure of the second component comprises a higher rate of voids (i.e. the sum of the total volume of the voids over the total volume that structure has or the amount of surface content of either the inner or outer surface over the total inner or outer surface, respectively) than the first component.

The tubular first component being relatively compact aims at guiding a fluid through its interior without relevant losses of that fluid by leaking through the wall of the tubular first component.

Preferably, the structure of the first component is fully compact. This means that the structure respectively the tubular wall (particularly the inner surface of that wall) representing the first component comprises no relevant voids or at least no voids that lead to a leaking of a fluid through that structure. However, this does not exclude a possible diffusion of individual constituents of the fluid into and through that structure.

The less compact structure of the second component may provide a relative high flexibility, allowing the implant to conform to surrounding tissue, thereby improving the fit of the medical implant. Additionally, the less compact structure of the second component, which may provide a plurality of voids and in particular through-holes, may be beneficial regarding an ingrowth of the implant into surrounding tissue, thereby leading at least to a secure positioning of the implant.

The combination of a Bombyx mori fibroin-based tubular first component and a less compact second component may therefore result in a mechanically strong and/or resilient, flexible or even rigid medical implant suitable for a wide range of applications, particularly long-term or permanent implantation procedures, most preferably for procedures with endoluminal access or for endoluminal applications or bridging.

The second component of the medical implant can be made of an absorbable or non-absorbable material. The choice of material for the second component can be selected based on the specific requirements of the medical application in which the implant is to be used.

Absorbable materials are materials that are gradually fully or mostly absorbed by a body over time. These materials can be advantageous in situations where the implant is intended to provide temporary support or reinforcement to a tissue or organ and can be gradually replaced by the body's own tissue as it heals. Non-absorbable materials, on the other hand, are materials that are not absorbed by the body and may remain in place indefinitely over the patient's lifetime. These materials can be advantageous in situations where the implant is intended to provide permanent support or reinforcement to a tissue or organ. The use of either absorbable or non-absorbable materials for the second component provides the medical implant with additional flexibility in terms of its potential applications. This allows the implant to be tailored to the specific needs of the patient and the medical procedure in which it is to be used.

In the case, when the second component of the medical implant is at least partially made of an absorbable material, it can preferably be made partially or entirely of Bombyx mori fibroin. The use of fibroin in both the first and second components of the medical implant can provide additional benefits, such as improved compatibility with the body's tissues and a reduced risk of adverse immune responses. This can help to improve the safety and effectiveness of the implant when used in medical procedures.

In a preferred embodiment of the medical implant, the second component is provided as a support structure that mechanically supports the first component. This support structure can provide additional strength and rigidity to the implant, helping to maintain its shape and structural integrity when implanted within the body. In particular, the second component, as a support structure, may be designed to support the first component against inflation (i.e. a decrease of its cross-section) and/or against deflation (i.e. an increase of its cross-section) and/or against buckling, i.e. kinking.

The support structure can be designed in a variety of ways, depending on the specific requirements of the medical application in which the implant is to be used. For example, the support structure can be a solid respectively fully compact structure, or it can be a lattice or mesh-like structure. The second component may, for example, surrounds the first component completely or partially. The support structure can also be integrated with the first component, such as by being embedded within the fibroin material of the first component. The use of a support structure can provide the medical implant with additional mechanical stability, helping to ensure that the implant maintains its shape and position within the body. This can be particularly important in applications where the implant is subjected to physical stresses or movements, such as in orthopaedic, visceral, urological or cardiovascular applications. The support structure can also help to protect the first component from damage, such as from mechanical impacts or abrasion, thereby helping to prolong the lifespan of the implant.

In a preferred embodiment, the second component may have a plastically deformable structure, in particular by being made of at least one plastically deformable material. This will allow to bring the implant into a desired shape, which is particularly suited for a specific medical use.

In some embodiments, the second component of the medical implant is made of or comprises at least one metal. The use of metal in the second component can provide the implant with additional strength and durability, making it particularly suitable for use in applications where the implant is subjected to high levels of physical stress or strain. The metal used in the second component can be selected from a wide range of biocompatible metals, depending on the specific requirements of the medical application in which the implant is to be used. In some cases, the second component may comprise a metal alloy or a composite material that includes metal. These materials can provide a balance of properties, such as strength, flexibility, and biocompatibility, tailored to the specific needs of the medical application.

In certain embodiments, the second component of the medical implant is made of or comprises one or more specific metals, including absorbable metallic magnesium, zinc, iron, molybdenum, and its alloys or non-absorbable metallic materials such as but not limited to titanium, stainless steel and nickel or nickel-titanium (nitinol) or its alloys. These metals are known for their biocompatibility and mechanical properties, making them suitable for use in absorbable, partially absorbable or non-absorbable medical implants.

Magnesium, zinc, iron and molybdenum are particularly interesting choices as they are absorbable metals. This means that over time, these metals can safely degrade within the body, eliminating the need for a second surgery to remove the implant, or in the case of partially absorbable implants fulfilling other functions such as releasing additional space for tissue to grow in. This can be particularly beneficial in applications where the implant is intended to provide temporary support or reinforcement to a tissue or organ and can be gradually replaced by the body's own tissue as it heals.

Titanium, stainless steel and nitinol, on the other hand, are known for their high strength and corrosion resistance, making them ideal for use in applications where the implant is intended to provide permanent support or reinforcement to a tissue or organ.

Molybdenum is a trace element that is essential for human health, and its inclusion in the second component can provide additional benefits, such as improved biocompatibility and reduced risk of adverse immune responses.

The use of these specific metals in the second component can provide the medical implant with additional mechanical stability, resilience or adoptability as well as biocompatibility, and in some cases, biodegradability, helping to improve the safety and effectiveness of the implant when used in medical procedures.

In some embodiments, the second component of the medical implant comprises an oxide surface layer, which is made of or comprises at least one oxide. This oxide surface layer can be naturally occurring, such as the passivation layer that forms on the surface of metals like titanium, stainless steel or nitinol when exposed to air, i.e. oxygen, or bodily fluids, or it can be artificially created through processes such as anodization, plasma-electrolytic oxidation (PEO), also known as micro-arc oxidation (MAO), or thermal oxidation.

The oxide surface layer can provide several benefits to the medical implant. Firstly, it can enhance the biocompatibility of the implant by providing a stable, inert surface that minimizes the potential for adverse reactions with the body's tissues. This can help to reduce the risk of complications such as inflammation or infection following implantation.

Secondly, the oxide surface layer can improve the corrosion resistance of the implant. This is particularly important for implants made (partially) from metals, as it can help to prevent the release of metal ions into the body, which could potentially cause adverse reactions.

Thirdly, the oxide surface layer can enhance the mechanical stability of the implant by providing a hard, wear-resistant surface. This can be particularly beneficial in applications where the implant is subjected to high levels of physical stress or strain.

Fourthly, the oxide surface layer can also provide a suitable surface for the attachment and growth of cells, which can aid in the integration of the implant with the surrounding tissue. This can help to improve the long-term stability and performance of the implant within the body.

Finally, the oxide layer may have a significant effect on absorbability of the second component, if applicable, e.g. by slowing down or accelerating degradation, i.e. decreasing or increasing corrosion rate. Thus, by providing a specific oxide layer a specific absorbability of the second component can be achieved.

The second component of the medical implant can have various structural configurations, including a textile structure, a chaotic structure, a helical (coil-like) structure, and/or a meshed structure. These structures can provide the implant with additional flexibility and/or resilience and/or plastic deformability (adaptability), allowing it to conform more closely to the shape of the surrounding tissue or the luminal structure to be supported or replaced, and can optionally also enhance the mechanical properties of the implant. Further, those specific structures of the second component may enhance an ingrowth of the implant into surrounding tissue.

A textile structure refers to a structure that is spun, twisted, carded, drawn, waved, knitted, or braided, similar to a fabric. This type of structure can provide the implant with a high degree of flexibility and conformability, allowing it to adapt to the shape of the surrounding tissue. It can also provide the implant with a high surface area, which can enhance the interaction between the implant and the body's tissues.

A chaotic structure refers to a structure that lacks a regular or predictable pattern. This type of structure can provide the implant with a high degree of flexibility and adaptability, allowing it to conform to the shape of the surrounding tissue or the luminal structure to be supported or replaced. It can also provide the implant with a large surface area, which can enhance the interaction between the implant and the body's tissues as well as with the first or any additional component of the implant.

A helical structure refers to a structure that is shaped like a helix or spiral, in particular like a helical spring or coil. This type of structure can provide the implant with a high degree of flexibility and resilience, allowing it to withstand the physical stresses that may be encountered within the body to a certain degree, in particular buckling or kinking.

A meshed structure refers to a structure that is made up of a network of interconnected filaments or fibres or to a structure that is made up of geometrically defined or chaotic voids within a formerly closed structure. This type of structure can provide the implant with a high degree of flexibility and conformability, allowing it to adapt to the shape of the surrounding tissue or the luminal structure to be supported or replaced. It can also provide the implant with a large surface area, which can enhance the interaction between the implant and the body's tissues as well as the first or any additional component of the implant.

A meshed structure which is made up of geometrically defined or chaotic voids within a formerly closed structures can preferably be produced by subtractive production methods, i.e. milling or laser cutting, starting from a closed structure such as a tube or mandrel.

The use of these structural configurations in the second component can provide the medical implant with additional mechanical stability and flexibility, helping to ensure that the implant maintains its shape and position within the body. Additionally, those structural configurations, while having sufficient stability, have a rather low compactness, which will lead to a low weight of the implant as well as a good ingrowth of the implant into surrounding tissue or decreased degradation by-products as well as degradation time in case of absorbable or partially absorbable implants.

The second component of the medical implant can be positioned in various ways relative to the first component. Specifically, the second component can contact an outer surface of the first component, an inner surface of the first component, or it can be embedded into the first component.

When the second component contacts the outer surface of the first component, it will not interfere with the flow of a fluid through the tubular first component. Further, it may enhance the ingrowth of the implant into surrounding tissue.

Having the second component contacting the inner surface of the first component may allow to achieve a non-interference with the surrounding tissue, which may be beneficial in some applications. It may also help endoluminal tissue such as the endothelium (inner wall of a vessel) to form a cell layer on the rather porous surface of the second component and hence building another internal smooth layer of cells which promotes hemocompatibility and decreases thrombogenicity.

Having the second component being embedded into the first component may allow to achieve a non-interference with both, a fluid within the tubular first component and the surrounding tissue, which may be beneficial in some applications. For example, it may improve the mechanical properties of the overall device by promoting mechanical strength, compliance, resilience or adaptability.

In some embodiments, the first component of the medical implant is partially made of collagen and/or elastin (in addition to at least the fibroin). These are natural proteins found in the body's tissues, known for their biocompatibility and unique mechanical properties. Collagen is the most abundant protein in the body, providing structural support to various tissues including skin, tendons, ligaments, and bones. It is characterized by its high tensile strength and flexibility. Incorporating collagen into the first component of the implant can enhance its biocompatibility and mechanical strength, making it particularly suitable for use in applications where the implant is intended to provide support or reinforcement to a tissue or organ.

Elastin, on the other hand, is a protein that provides elasticity to tissues, allowing them to return to their original shape after stretching or contracting. Incorporating elastin into the first component of the implant can enhance its flexibility and resilience, making it particularly suitable for use in applications where the implant needs to withstand repeated stretching or bending, such as in cardiovascular or orthopaedic applications.

The first component of the medical implant, which is tubular in shape, can have a wall thickness that falls within a specific range. This wall thickness can be between 5 $\mu$m and 1000 $\mu$m, or more specifically, between 30 $\mu$m and 600 $\mu$m, or even more specifically, between 50 $\mu$m and 500 $\mu$m. Other preferred ranges such as between 50 $\mu$m and 150 $\mu$m, between 150 $\mu$m and 250 $\mu$m, between 250 $\mu$m and 350 $\mu$m, between 350 $\mu$m and 450 $\mu$m, between 450 $\mu$m and 550 $\mu$m, between 550 $\mu$m and 650 $\mu$m as well as between 650 $\mu$m and 750 $\mu$m may also be beneficial.

The wall thickness of the first component can significantly influence the mechanical properties of the implant, including its strength, flexibility, and resilience. A thinner wall can provide the implant with greater flexibility, allowing it to conform more closely to the shape of the surrounding tissue. However, if the wall is too thin, it may not provide sufficient strength or durability, particularly in applications where the implant is subjected to high levels of physical stress or strain.

Conversely, a thicker wall can provide the implant with greater strength and durability, making it particularly suitable for use in applications where the implant is intended to provide substantial support or reinforcement to a tissue or organ. However, if the wall is too thick, it may reduce the flexibility of the implant, potentially making it less comfortable or less effective in certain applications.

The first component of the medical implant, which is tubular in shape, can have various geometrical configurations. Specifically, the first component can be conical in shape, or it can comprise at least one cylindrical section and at least one of: a conical first end, a conical second end, or a conical middle section which may be positioned between cylindrical sections.

A conical shape can provide the first component with a tapered profile, which can be advantageous in certain medical applications. For example, a conical shape can facilitate the insertion and positioning of the implant within the body, particularly in applications where the implant needs to be inserted into a narrow or constricted space or connected to another luminal structure (e.g. vessel or hollow organ) with the same or a similar diameter of the respective cylindrical part of the two entities to be connected (implant and the tissue).

A cylindrical section, on the other hand, can provide the first component with a uniform diameter along its length. This can be advantageous in applications where the implant needs to provide consistent support or reinforcement along its entire length or allow for minimally obstructed flow of bodily fluids (e.g. blood).

The inclusion of a conical first end, a conical second end, or a conical middle section can provide the first component with a tapered profile at one or more specific locations along its length. This can be advantageous in applications where the implant needs to conform to a specific shape or contour within the body.

The first component of the medical implant, which is tubular in shape, can have at least one section with a round or ellipsoidal cross-section. This cross-sectional shape of the first component can significantly influence the mechanical properties of the implant, including its strength, flexibility, and resilience. The (largest) diameter of the cross-section is preferably between 4 mm and 30 mm. Other possibilities include a diameter between 0.1 mm and 1 mm, between 1 mm and 4 mm, between 4 mm and 6 mm, between 6 mm and 10 mm, between 10 mm and 20 mm, between 20 mm and 30 mm, and between 30 mm and 50 mm.

A round cross-section can provide the first component with a high degree of symmetry, which can enhance its mechanical strength and stability. This can be particularly beneficial in applications where the implant is subjected to high levels of physical stress or strain, such as in orthopaedic or cardiovascular applications.

An ellipsoidal cross-section, on the other hand, can provide the first component with a higher degree of flexibility in one direction compared to the other. This can be particularly beneficial in applications where the implant needs to conform to a specific shape or contour within the body, or where the implant needs to flex or bend in a specific direction.

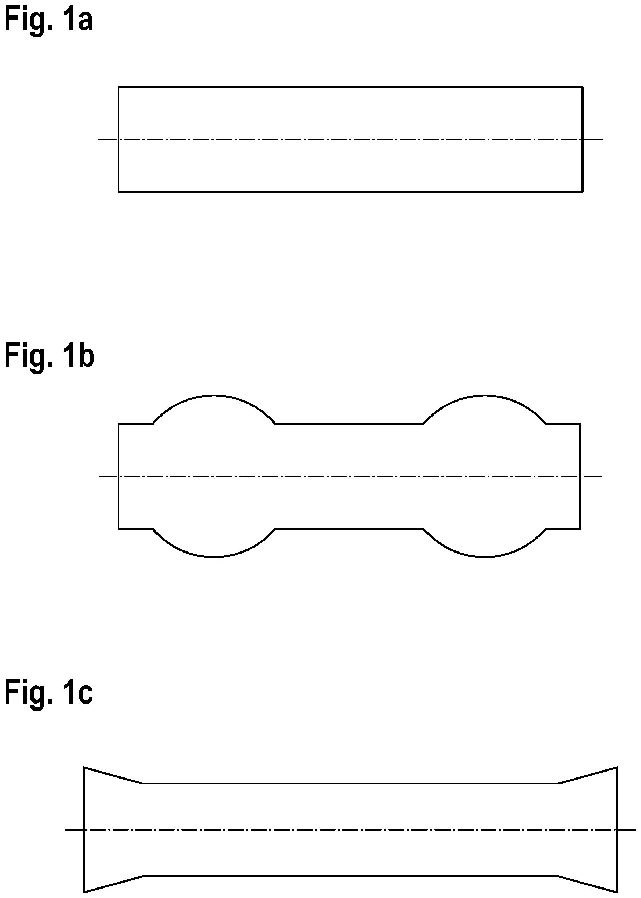

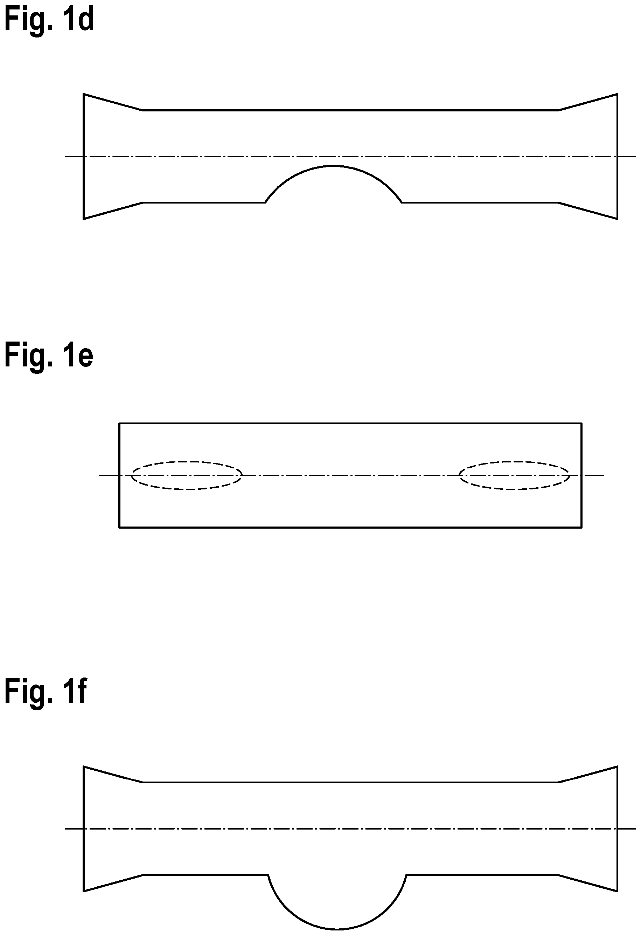

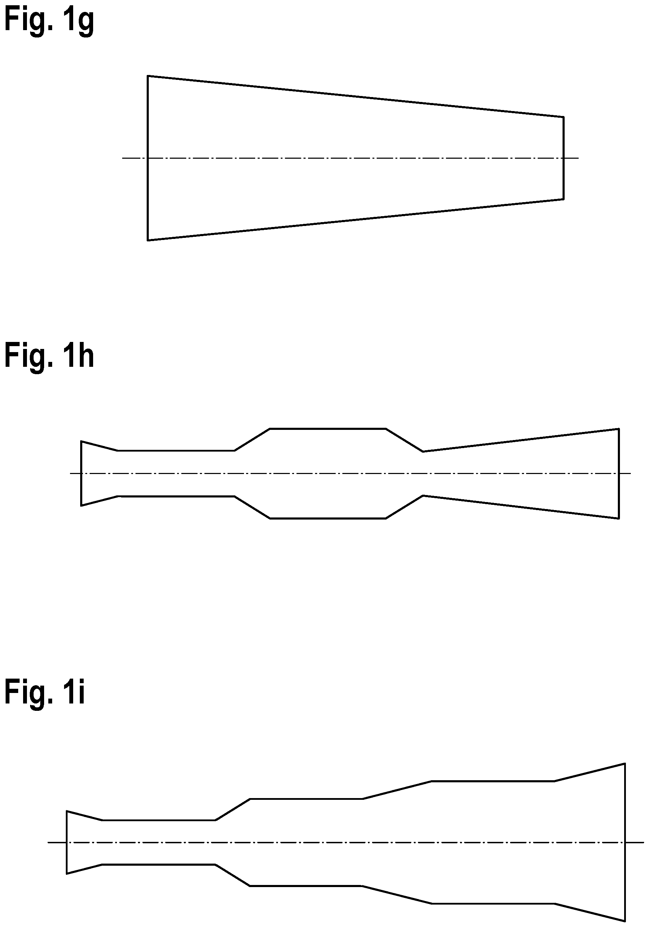

Further asymmetrical variations in the cross-section and/or the longitudinal form include concave or convex parts. These variations may include inward or outward bulging from the round or ellipsoidal cross-section. This can be particularly beneficial in applications where the implants need to adapt to a patient-specific shape. Examples of these variations are shown in Fig. 1a to 1i.

The tubular structure of an implant according to the invention may further include oriented and/or non-oriented pores or openings which can act as inlets or outlets for fluids and media. These pores or openings can be particularly beneficial in case of a presence of side branches originated from the diseased tubular structures as in the case of brain vessels or for enhancing the fluid flow through the structures as in the case of ureteral stents.

In some embodiments, the medical implant may further comprise a tubular third component made partially or entirely of elastin, whereas the third component represents an inside, in particular the full inside of the implant. The third component may therefore be the only component intended for contact with a body fluid to be guided through the implant. This embodiment makes use of an advantageous behaviour of elastin when contacted with some body fluids, in particular blood.

In some embodiments, the medical implant may further comprise a tubular third component made partially or entirely of magnesium and/or zinc particles, whereas the third component represents an inside, in particular the full inside of the implant. The third component may be the only component intended for contact with bone to guide a periosteal regeneration. This embodiment makes use of an advantageous behaviour of the metal particles in the process of bone and periosteal regeneration regarding the mechanical properties.

In a preferred embodiment, the third component may be provided as a coating of the inside surface of the first component and/or the second component.

The invention also encompasses a method for producing the medical implant, wherein at least a section of the first component is made by dip-coating. Dip-coating is a process that involves immersing a substrate into a liquid coating material, then withdrawing it at a controlled speed. As the substrate is withdrawn, the coating material adheres to the surface of the substrate, forming a thin, uniform layer.

In the context of the present invention, the substrate could be a mould or a mandrel shaped to the desired dimensions of the first component of the medical implant. The coating material would be a solution or suspension of isolated fibroin from Bombyx mori, possibly in combination with other materials such as collagen or elastin. After the substrate is dipped into the coating material and withdrawn, the coating is allowed to dry or is cured, solidifying into a tubular structure that forms the first component of the medical implant. The curing of the obtained structure may be optimized on the basis of the specific structure with processes that include drying at room temperature, use of high temperatures in an oven up to 250°C, vacuum drying, vacuum drying in high humidity environments, exposure to alcohols or water vapor, heat convection, exposure to infrared or ultraviolet light, especially in the case in which chemical modifications and photopolymers are used. The curing procedure can be used both for intermediate drying between different components and layers or for the final drying.

The dip-coating process allows for precise control over the thickness and uniformity of the coating, which in turn determines the wall thickness of the first component of the medical implant. In the dip-coating procedure, the control of the single-layer thickness is determined by the specific withdrawal speed and by the viscosity of the solution used according to the Landau-Levich equation. Furthermore, the dip-coating process allows to incorporate a plurality of layers and to tune the thickness according to the number of layers deposited. This process also allows for the production of complex shapes and structures, such as conical or cylindrical sections, or sections with round or ellipsoidal cross-sections, as described previously.

The dip-coating process is also highly scalable, making it suitable for the mass production of medical implants. Furthermore, because the process involves the use of a liquid coating material, it is possible to easily incorporate additional components or additives into the coating, such as drugs or other bioactive agents, which can be released from the implant over time, or functional additives such as photopolymers to add beneficial properties such as a light curing property during the production of the application of the medical implant in patients. This adds another layer of functionality to the medical implant, making it not just a structural support, but also a vehicle for targeted drug delivery.

The method for producing the medical implant can also involve specific techniques for creating the second component with a chaotic structure or a textile structure, for example, but not limited to, adding additives which form pores, for example by evaporation, or other mechanisms to shape and dress the second component.

For creating a chaotic structure, electrospinning can be employed. Electrospinning is a fibre production method which uses electric force to draw charged threads of a material solutions or material melt up to fibre diameters in the order of some hundred nanometres. Electrospinning can create non-woven structures of extremely fine fibres with high porosity and large surface area, which can be ideal for the chaotic structure of the second component.

For creating a textile structure, spinning, twisting, carding, fibre drawing, weaving, knitting, or braiding fabric production techniques can be used. Spinning, twisting, carding and fibre drawing refer to the preparation steps of the filament before being able to produce a fabric. Carding involves separating and aligning the fibres to produce a loose, fluffy web, or sliver. In the drawing step, the sliver is stretched and combined with others to improve uniformity and align the fibres. The fibres can be then twisted in a thinner strand which is then stretched in the spinning step to form the yarn or thread. Knitting, weaving, and braiding are traditional textile manufacturing methods that interlace yarns to create a fabric with specific properties. Knitting involves forming a series of interconnected loops from one or more yarns, weaving is the interlacing of two sets of yarns, and braiding involves intertwining three or more yarns to create a flat or tubular fabric. Weaving, knitting and braiding can create flexible, porous and thus low-compact structures that can be ideal for the textile structure of the second component.

These techniques allow for the creation of complex structures with specific mechanical and physical properties, making them suitable for use in the second component of the medical implant. Furthermore, these techniques are highly versatile and can be adapted to use with a wide range of materials, including the fibroin from Bombyx mori, as well as other biocompatible and biodegradable materials.

The method for producing the medical implant can involve a specific technique for creating the optional oxide surface layer on the second component. Specifically, Plasma Electrolytic Oxidation (PEO) can be employed to create the oxide surface layer. PEO, also known as micro-arc oxidation or anodic spark deposition, is an electrochemical surface treatment process used to produce oxide coatings on absorbable and non-absorbable metals. It is done by applying a high voltage to the metal, causing an intense anodic spark to form. This spark generates a high local temperature, leading to the formation of an oxide layer on the surface of the metal. The PEO process can create a hard, wear-resistant oxide surface layer on the second component of the medical implant. This oxide layer can enhance the mechanical stability of the implant, protect the underlying metal from corrosion, and improve the biocompatibility of the implant. The thickness and properties of the oxide layer can be controlled by adjusting the parameters of the PEO process, such as the voltage, current density, and treatment time.

The method for producing the medical implant can involve a specific technique for the formation of a passivation layer on the second component. Specifically, the immersion of the second component in a solution, such as for example body fluids, can lead to the formation of a passivation layer without the use of any high voltage current. Metals as aluminium, titanium or stainless steel naturally form a passivation layer when exposed to the oxygen contained in air or water thus increasing the corrosion resistance.

The medical implant as described can be used in a variety of medical applications, serving as a stent, patch, or graft in various parts of the body.

As a biliary stent or graft, the implant can be used to maintain the open passage of the bile duct, which can be blocked due to conditions such as gallstones or tumours. The implant can provide a conduit for bile to flow from the liver to the small intestine, aiding in digestion.

The biliary stent or graft may be characterized by a conical or a cylindrical section and may comprise flared ends to avoid the migration of the device. The conical shape may be favourable to connect the bile duct in case of liver transplant and to adapt for possible different dimensions between the donor and the recipient. The biliary stent or graft can be delivered both in a minimal invasive procedure in the case of a biliary stricture or in an open surgery in the case of the liver transplant. The biliary stent or graft is therefore either deployed in presence of the stricture or sutured to the vessel wall in the case of the liver transplant.

As a urethral or ureter stent or graft, the implant can be used to ensure the open passage of urine from the kidneys to the bladder, and from the bladder to the outside of the body. This can be particularly useful in cases where the urethra or ureter is blocked or narrowed due to conditions such as kidney stones or tumours. In this case, the ureteral or urethral stents or grafts may be characterized by flared ends or pigtails at both the extremities to avoid the migration of the devices. The ureteral or urethral stents or grafts further comprise the presence of longitudinal oriented and non-oriented pores or openings to enhance the urine flow to the bladder and to the outside of the body. These stents or grafts are delivered endoscopically and smoothness is required on the external surface to decrease the friction between the ureter or urethra wall and the tubular structure. The urethral or ureteral stents of grafts may comprise a full polymeric structure combining two different manufacturing techniques to achieve the compact and less compact components, or they can include a polymeric textile structure, or they can include a metallic coiled reinforcement.

As an oesophageal stent or graft, the implant can be used to maintain the open passage of the oesophagus, which can be narrowed due to conditions such as oesophageal cancer or strictures. The implant can provide a conduit for food and liquid to pass from the mouth to the stomach. The oesophageal stent or graft may be characterized by the combination of polymeric and a metallic component in order to improve the radial force and maintain the patency of the lumen. The oesophageal stent or graft may also comprise flared ends to avoid the migration of the device. The device may be delivered endoscopically and it is therefore deployed in correspondence of the oesophageal stenosis.

As a vascular graft or stent, in particular a coronary or small diameter stent or graft, the implant can be used to maintain the open passage of blood vessels, which can be narrowed or blocked due to conditions such as atherosclerosis. The implant can provide a conduit for blood to flow, ensuring the delivery of oxygen and nutrients to various parts of the body. A vascular graft may have a compact cylindrical, hollow shape which avoids leakage of the structure and allows the suturability of the graft to the vessel ensuring the formation of a bypass. A vascular stent may have a porous metallic or polymeric structure which could be complemented by a compact polymeric tubular structure forming a stent-graft. In this configuration a metallic or polymeric mesh could be laser-cut, braided, knitted or weaved and it may be deployed through a specific delivery system allowing for the self-expansion or the balloon expansion of the metallic mesh.

As a duodenal, renal, hepatic, venous, or brain stent or graft, the implant can be used to maintain the open passage of various other ducts, vessels, or channels within the body, which can be narrowed or blocked due to various conditions. The shape of these stents and grafts needs to adapt to the shape of the specific vessels allowing also for specific openings in correspondence of the presence of side-branches as may happen in the case of brain stents or grafts. These stents or grafts may be reinforced by a polymeric or metallic support structure depending on the size and on the specific mechanical properties of the system. These stents or grafts may be delivered either endoluminally or through open surgeries.

In particular, as a brain stent or graft, and specifically as a flow diverter, the implant can be used to divert blood flow away from an aneurysm in the brain, reducing the risk of rupture and subsequent haemorrhagic stroke. In this configuration, the tubular structure described may present a metallic braided or knitted second component which is complemented by a polymeric graft structure able to divert the blood flow from the aneurysm. The graft structure may also present some discontinuities to allow for the blood flow towards side branches. The device may be delivered endoluminally through minimally invasive surgeries.

As a nerve graft, the implant can be used to reconnect the two ends of a nerve which may have been separated due to traumas or tumours. The nerve graft may therefore act as a bridge between the two ends allowing for the regrowth of the tissue. In this application, the tubular structure described may act as an outer shell for a sponge with aligned pores which is able to guide the regeneration of the tissue. The sponge could be realized through the freeze-drying of bioabsorbable biomaterials. The graft may have a cylindrical compact shape which allows for the suturing of the graft to the two ends of the original nerve. The graft may be delivered through an open or minimally invasive surgery but are not preferably delivered endoluminally.

As a suture-less anastomosis device, the implant can be used endoluminally to enhance the connection of an arterial vessel avoiding the necessity of multiple sutures. These devices can have both a conical or a cylindrical longitudinal section and could present outward or inward bulging or flared-ends at the extremities to allow for the fixing of the device to the vessel walls. The device may be delivered in an open surgery.

As a periosteal patch, the device can be used to regenerate the periosteum surrounding the bones which has applications in maxillofacial and orthopaedic surgery for bone regeneration, cartilage repair, implant integration and fracture repair. In this application, the periosteal patch may be applied in form of a membrane which is wrapped around the defect assuming then a tubular shape. The periosteal patch may therefore have a shape that is extremely adaptable to the shape of the defect treated. On the inner side the periosteal patch may comprise a third component made of magnesium and/or zinc particles in order to increase the mechanical properties and promote the bone regeneration, The periosteal patch may be realized in form of a membrane that is then flexible and can be plasticized to be adapted to the specific shape or may be produced in form of tubular structures that is then cut longitudinally and open to include the defect in treatment. The periosteal patch may be delivered both in form of minimally invasive or open surgery.

As a tendon graft, the device can be used to reconnect the two ends of a tendon ruptured for traumatic or pathological causes. The implant can provide the suturing and the reconnection of the two ends facilitating the regeneration of the tissue. In a preferred embodiment, this implant may be used for the reconstruction of the anterior cruciate ligament. The implant can be delivered through an open or minimally invasive surgery and needs to have a compact and mechanically strong structure to allow the suturing and the match with the mechanical properties of the original structure. The tubular structure may further be complemented with an inner sponge with aligned pores which could further guide the tissue regeneration and which could be obtained through a freeze-drying process of bioabsorbable biomaterials. The tendon graft may have a cylindrical shape with round or elliptical cross-sections.

The use of the medical implant in these various applications can provide significant benefits, including improved patient outcomes, reduced risk of complications, and improved quality of life for patients.

EXEMPLARY EMBODIMENTS

Further exemplary embodiments of the invention will be explained below with reference to the figures.

BRIEF DESCRIPTION OF THE FIGURES

Fig. 1a - 1i: are graphic representations of examples of implant according to the invention having different forms.

Fig. 2: is a graphic representation of a method used for sample preparation according to a first embodiment.

Fig. 3a: is a graphic representation an interpenetrated double-crosslinked network in which silk fibroin is physically crosslinked through β-sheets and ELRs are covalently linked through click chemistry.

Fig. 3b: is a graphic representation of a method used for sample preparation according to a second embodiment.

Fig. 3c: is a graphic representation of a method used for sample preparation of a graft made of silk fibroin, produced to be compared to the samples according to the second embodiment

Fig. 4:        is a graphic representation of a method used for sample preparation according to a third embodiment.

## DETAILED DESCRIPTION OF THE EXEMPLARY EMBODIMENTS

In each of the following embodiments, fibroin is used to produce a medical implant 1 according to the invention. To process the fibroin, it is extracted from Bombyx mori silk cocoons. For this, a fibroin aqueous solution was obtained using PureSilk° technology (Fibrothelium GmbH, Aachen, Germany), enabling medical-grade quality production of silk on an industrial scale for a broad range of concentrations. The PureSilk° technology is disclosed in WO 2023/099382 A1, the content of which is incorporated into this specification in its entirety by reference. Briefly, fibroin was separated from sericin by degumming it in a hot alkali solution before dissolving it in a proprietary nontoxic solvent system based on Ajisawa's reagent. The dissolved silk fibroin was fully dialysed against VE water within 8 hours using tailored extraction processing. The fibroin concentration was adjusted to 20 wt% and stored at 4°C.

## FIRST EMBODIMENT

The fibroin obtained was processed to obtain a medical implant according to the invention having a bilayer structure exploiting the easy processability of silk fibroin.
As a reference, a monolayer structure was created as well.

## PRODUCTION

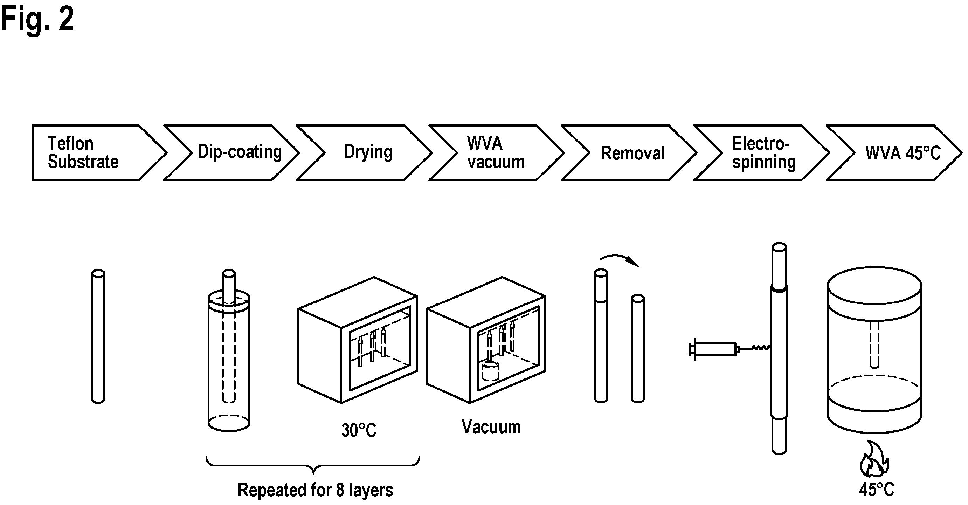

The entire production process is shown in Fig. 2. Teflon mandrels with a length of 120 mm and a diameter of 3 mm and 4 mm were selected, considering that internal biliary stents had a median size of 3.33 mm, with a range between 2.67 mm and 4 mm.
Two types of samples were produced whereby (i) monolayer devices were produced with dip-coating only and (ii) bilayer devices were produced using a combined dip-coating and electrospinning process. Before use, all the mandrels were rinsed with ethanol and water to ensure the absence of particles possibly affecting the fabrication procedure.

## MONOLAYER PRODUCTION

The first step consisted of the fabrication of the inner structure by multilayer dip-coating deposition. The entire procedure was performed on a numerically controlled setup that allowed reproducibility of the process, by maintaining a constant withdrawal velocity. The immersion and withdrawal velocities were constant at 5 mm/s and the immersion time was 30 s.
The fibroin solution used for the dip-coating procedure had a 20 wt% concentration and was mixed with glycerol in a proportion equal to the 30 % of fibroin content to induce the insolubility of silk fibroin and to improve the flexibility of the device. The dip-coating procedure was repeated 8 times with a drying process in between the layers performed at 30°C for 2 hours. The direction of coating was inverted after four layers to ensure a homogenous distribution of the dip-coating layer. After the last drying step, 20 mm of coating was removed from each extremity of the mandrel due to the incomplete immersion given by the inversion procedure.
To remove the coating structure from the mandrel, water vapor annealing was performed. Water vapor annealing (WVA) consists of the exposition of the samples to water vapor to alter the crystallinity of silk fibroin. The process was carried out at 25°C under vacuum inside a vacuum oven for three hours. Afterwards, the coating part could be easily pulled out of the substrate obtaining the entire tubular dip-coated layer.

## BILAYER PRODUCTION

To produce the bilayer devices, the dip-coated monolayer specimens (as a first component of the medical device) underwent an additional step, whereby an outer layer (as a second component of the medical device with a less compact structure) was produced through a nozzle-less electrospinning procedure performed on an electrospinning setup.
In brief, a rotating stainless-steel spinneret was placed inside a 21 wt% silk fibroin aqueous solution reservoir and connected to a gear motor while the dip-coated tube was placed on a rotating aluminium rod-collector (3 mm diameter, 300 mm length) at a distance of 240 mm from the tip of the spinneret. A high voltage difference of 45-55 kV was applied between the collector and the reservoir. Spinning was performed at room temperature for 30 minutes to enable a proper deposition of the electrospun layer. Since this last layer is soluble in water, the structure was crosslinked through a water vapor annealing process, whereby samples were exposed to water at 45°C for 30 minutes to ensure the formation of β-sheets in the electrospun layer.

MANUFACTURING REPRODUCIBILITY ASSESSMENT

To assess the reproducibility of the stents being prepared, the linear density (LD) was measured through a non-destructive method. The linear density was calculated as the ratio between the mass of the sample and its length (Equation 1), where the mass was measured using a precision scale.

$$LD \left[\frac{mg}{cm}\right] = \frac{weight \ [mg]}{length \ [cm]} \qquad \qquad \text{Equation 1}$$

To account for the different diameters of the samples and therefore for the different lateral surface areas of the substrates, a normalized value of linear density divided by the circumference was calculated (Equation 2).

$$Normalised \ LD \left[\frac{mg}{cm^2}\right] = \frac{weight \ [mg]}{length \ [cm] * \pi D_{substrate} \ [cm]} \qquad \qquad \text{Equation 2}$$

The normalized linear density was therefore calculated for each batch.

RESULTS

MONOLAYER AND BILAYER BILIARY STENT PRODUCTION

The dip-coating process was performed successfully. This technique was reliably reproducible, with the shape of the mandrel and the macroscopic analysis revealing a homogeneous structure without gradients in coating distribution.
The bilayer biliary stents that were produced through the combined dip-coated and electrospinning process further had a homogeneous appearance, and the two layers were well integrated, without any peeling effect evident between the dip-coated inner structure and the outer electrospun fleece. Furthermore, the electrospun outer layer was uniformly distributed across the stent structure without any gaps.

MANUFACTURING REPRODUCIBILITY ASSESSMENT

Results regarding the manufacturing reproducibility of both the monolayer and bilayer, in terms of the normalized LD, show that there was no statistical difference between the LD of the batches for the monolayer samples, with the average normalized LD for all batches being $15.10 \pm 1.37$ mg/cm$^2$. The normalized linear density for the two bilayer batches was also checked. Even in the presence of a second layer, the difference between the two batches remained not statistically significant with an average value of $21.27 \pm 2.35$ mg/cm$^2$. Thanks to the non-destructive method used, all the four batches of samples could be measured even before the electrospinning of two of them.

SECOND EMBODIMENT

A medical implant according to the second embodiment comprises a mixture of fibroin and elastin as first component, a PET knitted mesh as second component, and elastin alone as third component (SF+ELR+PET Grafts). As the elastin ingredient, Elastin-like recombinamers (ELRs) were used, which consisted of two different polymers chemically modified for "click"-crosslinking. The two recombinamers were chemically modified and groups to enhance cell binding and enzymatic degradation were introduced. Briefly, 700 mg of each variant were received lyophilized and dissolved in 7 mL of distilled water at 4°C.
Another medical implant according to the second embodiment comprises a first component comprising fibroin and a second component made of a PET knitted mesh (SF+PET Grafts).
A monolayer of silk fibroin alone was additionally realized for reference (SF Grafts).

SF GRAFTS

Teflon mandrels with a length of 80 mm and a diameter of 4 mm were selected for the fabrication of the silk fibroin-only grafts. The fibroin solution used for the dip-coating procedure of the grafts had a 20 wt% concentration and was mixed with glycerol (VWR, Radnor, Pennsylvania, USA) in a proportion equal to the 30 % of fibroin content to induce the insolubility of silk fibroin and to improve the flexibility of the device. The dip-coating procedure was repeated 10 times with a drying process in between the layers performed at 30°C for 2 hours. The direction of coating was inverted after 5 layers to ensure a homogenous distribution of the dip-coating layer. After the last drying step, 20 mm of coating was removed from each extremity of the substrate due to the incomplete immersion given by the inversion procedure.

To remove the coating structure from the substrate, water vapor annealing was performed. Water vapor annealing consists of the exposition of the samples to water vapor to alter the crystallinity of silk fibroin. The process was carried out at 25°C under vacuum inside a vacuum oven for 3 hours. Afterwards, the coating part could be easily pulled out of the substrate obtaining the entire tubular dip-coated layer.

SF, ELR AND PET GRAFTS

The same Teflon mandrels were selected also for these grafts. The inner part of the graft was composed by ELR only to exploit the hemocompatibility properties of elastin. This was obtained by immersing the substrates consequently in the two ELR recombinamers solutions. The PET mesh was introduced to increase the mechanical stability of the grafts and it was heat-set after the knitting process at 170°C for 5 minutes.

The entire procedure was performed on a numerically controlled setup that allowed reproducibility of the process, by maintaining a constant withdrawal velocity. The immersion and withdrawal velocities were constant at 5 mm/s, the immersion time was 30 s and the time between two consecutive immersions was 1 minute. This procedure was repeated three times with a drying process in between the layers performed at room temperature for 10 minutes. An inversion of the coating direction was performed after the first two double layers to ensure the homogeneity of the coating distribution. The entire procedure was performed while maintaining the two solutions in an ice batch to prevent the ELRs from turning into a gel.

After these first three double layers, each ELR solution was mixed with an equal amount of the silk fibroin solution obtained. Other 17 double layers were realized with these solutions according to the procedure described above with an inversion in the coating direction after the eighth double layer. In this step the PET mesh was also introduced to have a complete embedding of the less compact structure in the polymeric matrix. The number of layers deposited is chosen to balance the necessity of wall thickness with the limited processing time given by the instability of the ELRs. The samples were then stored in 70 v/v% ethanol for 3 days to ensure the insolubility of the materials. The grafts were then immersed in water and gently removed from the mandrels excluding at each extremity 20 mm because of the inhomogeneous structure given by the inversion procedure.

SF AND PET GRAFTS

The PET knitted meshes were heat-set in an oven at 170°C for 5 minutes to reset the residual stresses left in the polymeric material from the knitting process.

The same procedure shown for the SF grafts was used for these grafts with the exception that the PET mesh was added before the inversion of the direction of coating. The PET mesh was therefore embedded in the silk fibroin matrix.

To remove the coating structure from the substrate, water vapor annealing was performed. Water vapor annealing consists of the exposition of the samples to water vapor to alter the crystallinity of silk fibroin. The process was carried out at 25°C under vacuum inside a vacuum oven for 3 hours. Afterwards, the coating part could be easily pulled out of the substrate obtaining the entire tubular dip-coated layer.

MORPHOLOGICAL CHARACTERISATION

To assess the reproducibility of the grafts being prepared, the linear density (LD) was measured through a non-destructive method. The linear density was calculated as the ratio between the mass of the sample and its length (Equation 1 above), where the mass was measured using a precision scale (ABT 320-4M, Kern, Balingen, Germany).

RESULTS

GRAFT PRODUCTION AND MORPHOLOGY

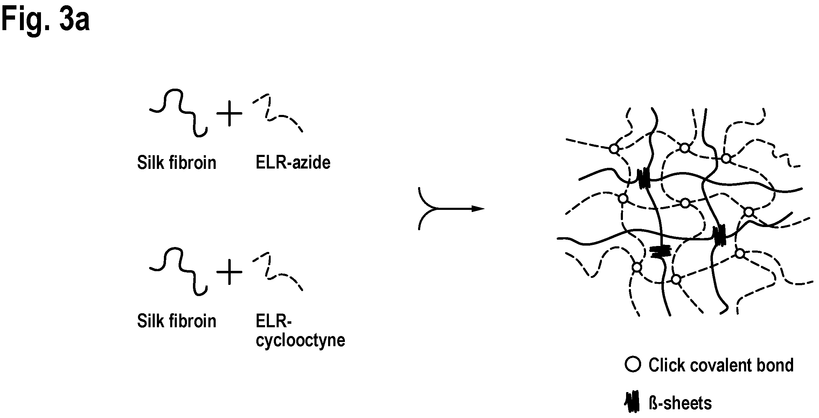

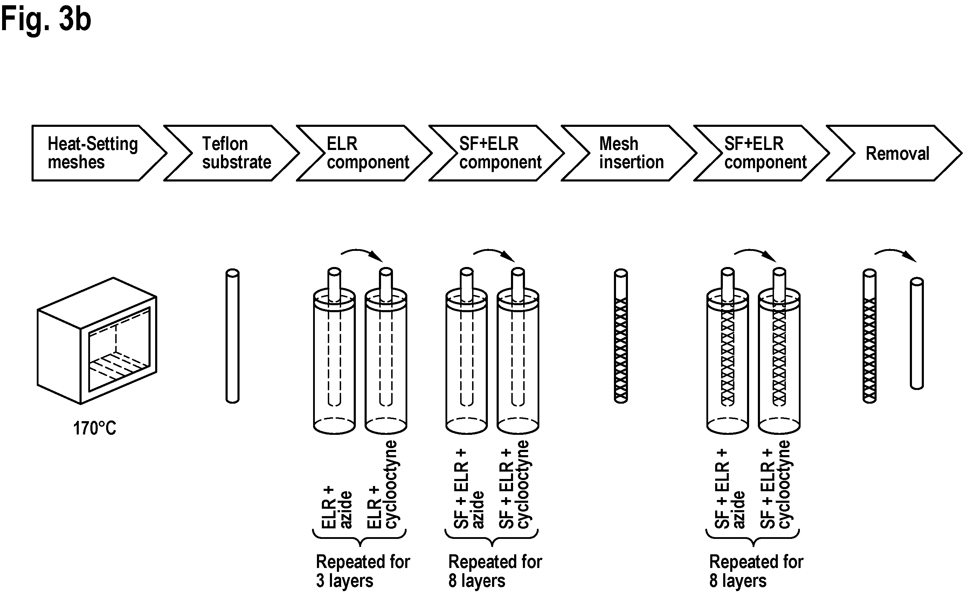

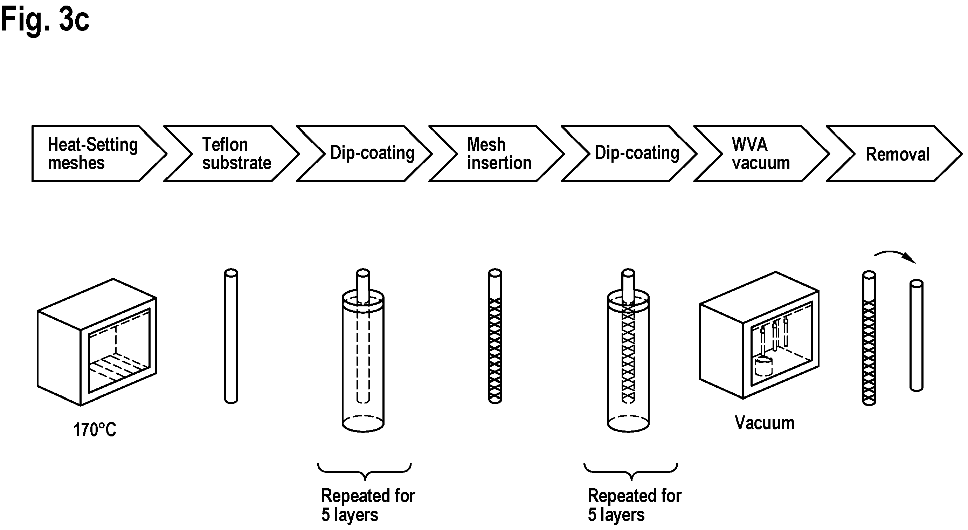

The dip-coating process was performed successfully for the silk fibroin grafts, the silk fibroin and elastin grafts and the silk fibroin and PET mesh grafts. In the case of the SF+ELR grafts the multilayer dip-coating deposition (Fig. 3b) allowed to create an interpenetrated double crosslinked network of ELR and silk fibroin (Fig. 3a) on the surface of the Teflon substrates, while in the case of the SF grafts a multilayer dip-coating monomaterial structure was formed (Fig. 3c).

The removal procedure from the Teflon substrates could be performed without damaging the graft prototypes. The procedure proved to be reproducible as shown by the linear density of the samples which is 33.50 $\pm$ 1.02 mg/cm for the SF+ELR+PET grafts and 20.86 $\pm$ 2.21 mg/cm for the SF grafts. The linear density of the SF+PET samples was 28.59 $\pm$ 1.20 mg/cm.

THIRD EMBODIMENT

The third embodiment represents a bioabsorbable medical implant, suitable as an oesophageal stent, that integrates a magnesium single-wire braided stent (second component) complemented with a dip-coated multilayer fibroin inner cover (first component). This design uses the metallic stent on the outside to avoid migration and promote good anchoring to the tissue, while having a continuous polymeric structure on the inside to avoid tissue hyperplasia and infiltration.

MATERIALS AND METHODS

PRODUCTION

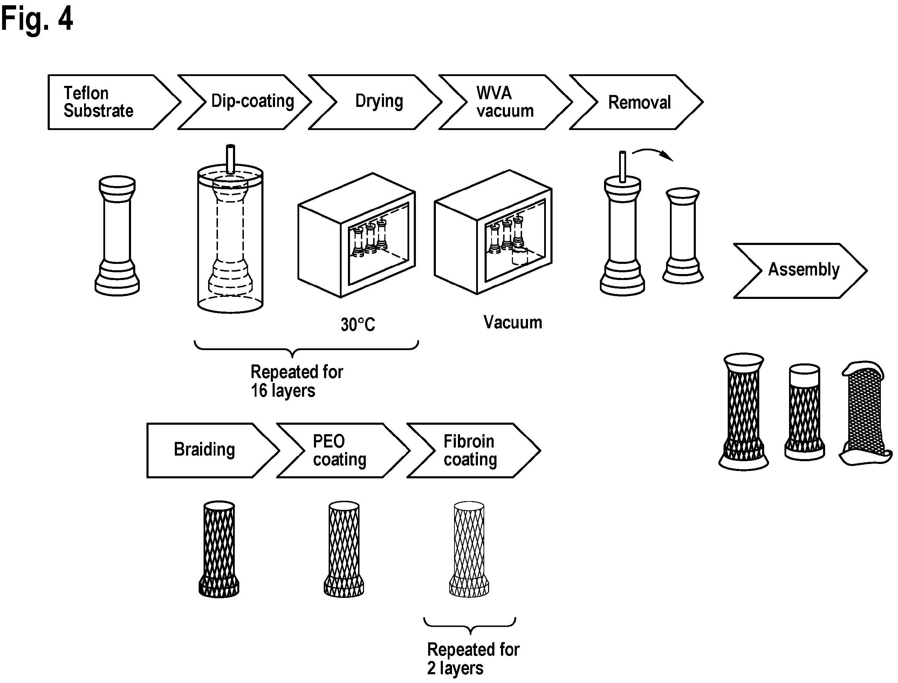

The entire production process is shown in Fig. 4. The stent structures were made of Magnesium WE43MEO with a wire of 0.3 mm diameter and presented an anti-migration flared or conical end. Teflon mandrels composed of three different parts were used. In the mandrel there were an anti-migration flared end on one side as in the stent braided structure and additional flares at each side to permit the folding over of the structure and the fixation of the tube to the stent. The diameter selected for both the stent and the Teflon mandrel was 23 mm for the core and 28 mm for the flared end and the length was 100 mm. The tubular silk coverings were fabricated using the multilayer dip-coating deposition disclosed in the embodiment 1 above.

METAL STENT

A WE43MEO magnesium wire with 0.3 mm diameter was manually braided in a stent structure presenting 12 crowns, with an approximate angle of 64°. To carry out this operation, a mandrel was used that enabled the stent structure to be braided correctly. The mandrel was provided with a flared end at one of the extremities to promote anchoring and inhibit migration.

Plasma electrolytic oxidation (PEO) was performed on the stent samples. PEO is a high-voltage anodising procedure that uses plasma discharges on the metallic sample surface leading to the formation of an outer porous film. A phosphate-based electrolyte was used together with a pulsed rectifier set to produce the outer PEO coating layer.

Since the PEO coating exhibits a porous morphological structure, a silk fibroin coating was added in the attempt to fill the pores and add an additional protective layer against magnesium corrosion. Two layers of silk fibroin coating were applied to the braided stent frames through dip-coating. For this, a silk fibroin solution is diluted to 2 wt% and mixed with glycerol in a proportion equal to the 30% of fibroin content to induce the insolubility of silk fibroin. Inversion of the coating direction was performed after the first layer to ensure the homogeneous distribution of silk fibroin. A drying process of 2 hours at 30°C was performed in between the layers. The procedure with the low concentration of silk fibroin ensured the absence of gap-filling protecting selectively the wire structure without affecting the flexibility.

INNER FIBROIN TUBE

The inner tube was fabricated using the multilayer dip-coating deposition process already described win connection with embodiment 1.

The fibroin solution used for the dip-coating procedure had a 20 wt% concentration and was mixed with glycerol in a proportion equal to the 30% of fibroin content to induce the insolubility of silk fibroin and to improve the flexibility of the device. The dip-coating procedure was repeated 16 times with a drying process in between the layers performed at 30°C for 2 hours. The direction of coating was inverted after 8 layers to ensure a homogenous distribution of the dip-coating layer. After the last drying step, 1 cm of coating was removed from each extremity of the substrate due to the incomplete immersion given by the inversion procedure. To remove the coating structure from the substrate, a water vapor annealing procedure was performed. The process was carried out at 25°C under vacuum inside a vacuum oven for three hours. After this process, the coating part could be easily removed from the substrate obtaining the entire tubular dip-coated layer.

ASSEMBLY

After the water vapor annealing procedure, the tube was inserted into the stent and the flared extremities were folded over the stent to ensure adequate integration between the frame and coverings components. The stability was further assured by the application of sutures between the tube and the stent.

REPRODUCIBILITY ASSESSMENT

To assess the reproducibility of the silk fibroin specimens being produced, the linear density (LD) was measured through a non-destructive method. The linear density was calculated as the ratio between the mass of the sample and its length (Equation 1 above), where the mass was measured using a precision scale.

The linear density was therefore calculated for each batch and the differences between the batches were analysed with a one-way ANOVA test investigating the dependence of the linear density on the production batch.

RESULTS

PRODUCTION

An ANOVA test revealed no significant difference among the batches of the fibroin tubes (p-value = 0.265). The average linear density found was 116.32 $\pm$ 36.6 mg/cm. As regards the thickness, the comparison between the average thickness obtained for the biliary stents according to embodiment 1 and for the oesophageal membranes with no statistically significant difference (p-value = 0.532). The average thickness value was 128.200 $\pm$ 21.464 $\mu$m.

**Claims**

1. A medical implant comprising a tubular first component, which is partially or entirely made of isolated Bombyx mori fibroin, and at least a second component, whereas the second component has a less compact structure compared to the first component.

2. The medical implant according to claim 1, **characterized in that** the second component is made of an absorbable or non-absorbable material.

3. The medical implant according to claim 2, **characterized in that** the second component is partially or completely made of fibroin.

4. The medical implant according to one of the preceding claims, **characterized in that** the second component is a support structure mechanically supporting the first component.

5. The medical implant according to one of the preceding claims, **characterized in that** the second component is made of or comprises at least one metal.

6. The medical implant according to claim 5, **characterized in that** the second component is made of or comprises magnesium and/or zinc and/or iron and/or molybdenum and/or titanium and/or nickel, each individually either in its pure form or as an alloy.

7. The medical implant according to claim 5 or 6, **characterized in that** the second component comprises an oxide surface layer.

8. The medical implant according to one or the preceding claims, **characterized in that** the second component has a textile structure and/or a chaotic structure and/or a helical structure and/or a meshed structure.

9. The medical implant according to one or the preceding claims, **characterized in that** the second component contacts an outer surface of the first component or an inner surface of the first component or is embedded into the first component.

10. The medical implant according to one of the proceeding claims, **characterized in that** the first component is partially made from collagen and/or elastin.

11. The medical implant according to one of the proceeding claims, **characterized by** a tubular third component made partially or entirely of elastin or magnesium and/or zinc particles, whereas the third component represents an inside of the of the implant.

12. A method for producing a medical implant according to one of the proceeding claims, **characterized in that** at least a section of the first component is made by dip-coating.

13. The method according to claim 12 for producing a medical implant according to claim 7 or any claim depending on claim 8, **characterized in that** the chaotic structure is made by electrospinning and/or the textile structure is made by spinning, twisting, carding, drawing, weaving, knitting and/or braiding.

14. The method according to claim 12 or 13 for producing a medical implant according to claim 7 or any claim depending on claim 6, **characterized in that** the surface layer is a passivation layer, preferably made by PEO.

15. Use of a medical implant according to one of the claims 1 to 11 as at least one of: biliary stent or graft; urethra stent or graft; ureter stent or graft; oesophageal stent or graft; vascular graft or stent; in particular a coronary or small diameter stent or graft; duodenal stent or graft; renal stent or graft; hepatic stent or graft; venous stent or graft; brain stent or graft, in particularly flow diverter; nerve graft or conduit; tendon and ligament graft or conduit; suture-less anastomosis device; periosteal replacement.

**Fig. 1a**

**Fig. 1b**

**Fig. 1c**

# Fig. 1d

# Fig. 1e

# Fig. 1f

# Fig. 1g

# Fig. 1h

# Fig. 1i

# Fig. 2

# Fig. 3a

Silk fibroin + ELR-azide

Silk fibroin + ELR-cyclooctyne

○ Click covalent bond

Ⅲ ß-sheets

# Fig. 3b

| Heat-Setting meshes | Teflon substrate | ELR component | SF+ELR component | Mesh insertion | SF+ELR component | Removal |

170°C

ELR + azide  ELR + cyclooctyne

SF + ELR + azide  SF + ELR + cyclooctyne

SF + ELR + azide  SF + ELR + cyclooctyne

Repeated for 3 layers

Repeated for 8 layers

Repeated for 8 layers

EP 4 782 016 A1

# Fig. 3c

Heat-Setting meshes → Teflon substrate → Dip-coating → Mesh insertion → Dip-coating → WVA vacuum → Removal

170°C

Repeated for 5 layers

Repeated for 5 layers

Vacuum

EP 4 782 016 A1

# Fig. 4

Teflon Substrate → Dip-coating → Drying → WVA vacuum → Removal

30°C

Vacuum

Repeated for 16 layers

Assembly

Braiding → PEO coating → Fibroin coating

Repeated for 2 layers

## EUROPEAN SEARCH REPORT

**Application Number**

EP 25 15 3704

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/054787 A1 (SHETE ABHIJIT PRAVIN [IN] ET AL) 20 February 2020 (2020-02-20) <br><br> * page 1, paragraph 0001 * <br> * page 2, paragraph 0017 - paragraph 0023 * <br> * page 3, paragraphs 0026, 0027 * <br> * example 1 * <br> * claims 1-3 * <br> ----- | 1-6, 8-10,12, 13,15 | INV. <br> A61L27/04 <br> A61L27/22 <br> A61L27/34 <br> A61L31/02 <br> A61L31/04 <br> A61L31/10 |
| X | WO 2020/109222 A1 (FIBROTHELIUM GMBH [DE]) 4 June 2020 (2020-06-04) <br> * page 1, line 4 - line 6 * <br> * page 4, line 31 - page 5, line 3 * <br> * page 5, line 22 - line 25 * <br> * page 8, line 23 - line 27 * <br> * page 9, line 23 - page 10, line 26 * <br> * page 11, line 15 - line 24 * <br> * example 1 * <br> ----- | 1-15 | |

**TECHNICAL FIELDS SEARCHED (IPC)**

A61L

The present search report has been drawn up for all claims

| Place of search | Date of completion of the search | Examiner |
|---|---|---|
| The Hague | 3 July 2025 | Dudás, Eszter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P4C01)

## ANNEX TO THE EUROPEAN SEARCH REPORT
## ON EUROPEAN PATENT APPLICATION NO.

EP 25 15 3704

This annex lists the patent family members relating to the patent documents cited in the above-mentioned European search report.
The members are as contained in the European Patent Office EDP file on
The European Patent Office is in no way liable for these particulars which are merely given for the purpose of information.

03-07-2025

| Patent document cited in search report | Publication date | Patent family member(s) | Publication date |
|---|---|---|---|
| US 2020054787 A1 | 20-02-2020 | EP 3600463 A1 | 05-02-2020 |
| | | US 2020054787 A1 | 20-02-2020 |
| | | WO 2018173074 A1 | 27-09-2018 |
| WO 2020109222 A1 | 04-06-2020 | DE 102018129658 A1 | 28-05-2020 |
| | | EP 3886928 A1 | 06-10-2021 |
| | | WO 2020109222 A1 | 04-06-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9808557 B2

- WO 2023099382 A1